# EUROPÄISCHE PATENTSCHRIFT

(11) **EP 0 832 640 B1**
(45) Veröffentlichungstag und Bekanntmachung des Hinweises auf die Patenterteilung: **30.04.2008**
(21) Anmeldenummer: 97115328.3
(22) Anmeldetag: 04.09.1997
(51) Int. Cl.: A61K 8/49, A61K 8/41, A61Q 5/10, D06P 1/32

(54) **Oxidationsfärbemittel zum Färben von Keratinfasern**
Oxidation dyeing composition for keratin fibres
Composition de teinture d'oxydation des fibres kératiniques

(30) Priorität: 13.09.1996 DE 19637371
(43) Veröffentlichungstag der Anmeldung: 01.04.1998
(73) Patentinhaber: Henkel Kommanditgesellschaft auf Aktien, 40589 Düsseldorf (DE)
(72) Erfinder: Meinigke, Bernd, Dr. Diplom-Chemiker, 51399 Burscheid (DE); Rose, David, Dr. Diplom-Chemiker, 40723 Hilden (DE); Akram, Mustafa, Dr. Diplom-Chemiker, 22457 Hamburg (DE)

(56) Entgegenhaltungen:
- WO-A-97/10799
- DE-A- 4 400 757
- GB-A- 2 260 135

## Beschreibung

Die Erfindung betrifft Oxidationsfärbemittel zum Färben von Keratinfasern, die spezielle Kuppler-Entwickler-Kombinationen enthalten.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Gute Oxidationsfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme und den Einfluß chemischer Reduktionsmittel, z. B. gegen Dauerwellflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein.

Als Entwicklerkomponenten werden beispielsweise primäre aromatische Amine mit einer weiteren, in para- oder ortho-Position befindlichen freien oder substituierten Hydroxy-oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind u.a. p-Toluylendiamin, p-Aminophenol, N,N-Bis-(2-hydroxyethyl)-p-phenylendiamin, 2-(2,5-Diaminophenoxy)-ethanol, 1-Phenyl-3-carboxyamido-4-amino-pyrazolon-5 und 4-Amino-3-methylphenol und 2,4,5,6-Tetraaminopyrimidin.

Als Kupplerkomponenten werden in der Regel m-Phenylendiaminderivate, Naphthole, Resorcin und Resorcinderivate, Pyrazolone, m-Aminophenole sowie Pyridin-Derivate verwendet. Als Kupplersubstanzen eignen sich insbesondere 1-Naphthol, Pyrogallol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlor-resorcin, 2-Chlor-6-methyl-3-aminophenol, 2-Methylresorcin, 2,6-Dihydroxypyridin und 2,6-Diaminopyridin.

Bezüglich weiterer Farbstoffkomponenten wird ausdrücklich auf die Colipa-Liste, herausgegeben vom Industrieverband Körperpflege und Waschmittel, Frankfurt, Bezug genommen.

In der Regel gelingt es nicht, mit Hilfe einer einzigen Kuppler/Entwicklerkombination zu natürlichen Farbnuancen zu kommen. In der Praxis ist daher meist eine Kombination verschiedener Entwicklerkomponenten und Kupplerkomponenten erforderlich, um eine natürlich wirkende Färbung zu erhalten. Es besteht daher ständig Bedarf an neuen, verbesserten Kuppler/Entwickler-Kombinationen. Dies trifft insbesondere auch auf den Rot-Bereich zu, wo die gängigen Farbstoffe häufig noch nicht ganz befriedigende Reibechtheiten, Egalisiervermögen und Kaltwell- und Waschechtheiten aufweisen.

Es war daher die Aufgabe der vorliegenden Erfindung, neue Kuppler/Entwickler-Kombinationen zu finden, die die an Oxidationsfarbstoffvorprodukte zu stellenden Anforderungen in besonderem Maße erfüllen.

Überraschenderweise wurde nun gefunden, daß spezielle Kombinationen aus an sich bekannten Kuppler- und Entwicklerkomponenten zu außerordentlich intensiven Färbungen vor allem im Rot- und Orangebereich führen, die sich u.a. durch besonders gute Licht-, Wasch- und Reibechtheiten sowie ein unerwartet hohes Egalisiervermögen auszeichnen.

Gegenstand der vorliegenden Erfindung sind daher Oxidationsfärbemittel zum Färben von Keratinfasern, insbesondere menschlichen Haaren, enthaltend mindestens eine Kupplerkomponente und mindestens eine Entwicklerkomponente in einem wasserhaltigen Träger, dadurch gekennzeichnet, daß
- die Kupplerkomponente ausgewählt ist aus den Verbindungen gemäß Formel (I), 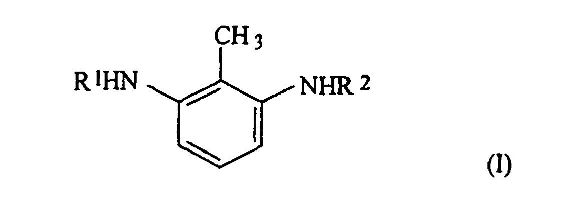 in der R¹ und R² unabhängig voneinander stehen für Wasserstoff, eine Mono- oder Dihydroxyalkylgruppe mit 2 bis 4 Kohlenstoffatomen oder eine Allylgruppe mit der Maßgabe, daß R¹ und R² nicht gleichzeitig Wasserstoff sind, und deren Salzen mit einer anorganischen oder organischen Säure und
- die Entwicklerkomponente gemäß Anspruch 1.

Unter Keratinfasern sind dabei Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Die erfindungsgemäßen Färbemittel enthalten als erste zwingende Komponente eine Verbindung gemäß Formel (I), die als Kupplerkomponente dient. Die Mehrzahl der Verbindungen gemäß Formel (I) sind Gegenstand der deutschen Patentschrift 41 32 615. Sowohl hinsichtlich der Herstellung der Verbindungen gemäß Formel (I) als auch hinsichtlich der einzelnen Substituentenkombinationen wird ausdrücklich auf den Inhalt dieser Druckschrift Bezug genommen. Diese Druckschrift offenbart zwar die Verwendung dieser Verbindungen als Kupplerkomponente in Haarfärbemittel in Kombination mit einer Reihe üblicher Entwicklerkomponenten. Die Druckschrift liefert jedoch keinerlei Hinweise auf die Verwendung dieser Kupplerkomponenten in Kombination mit den erfindungsgemäß beanspruchten, speziellen Entwicklerkomponenten.

Innerhalb der Verbindungen gemäß Formel (I) sind solche Substanzen bevorzugt, bei denen mindestens eine der beiden Gruppen R¹ und R² für eine 2-Hydroxyethylgruppe, eine 3-Hydroxypropylgruppe oder eine 2,3-Dihydroxypropylgruppe steht. Ebenfalls bevorzugt sind solche Substanzen, bei denen beide Gruppen R¹ und R² gleiche Gruppen, und zwar 2-Hydroxyethyl- oder 3-Hydroxypropylgruppen sind. 2,6-Bis(3'-hydroxypropylamino)-toluol und insbesondere 2,6-Bis(2'-hydroxyethylamino)-toluol sind daher ganz besonders geeignete Kupplerkomponenten gemäß Formel (I).

Die zweite zwingende Komponente der erfindungsgemäßen Färbemittel ist eine speziell ausgewählte Entwicklerkomponente. Eine erste, bevorzugte Entwicklerkomponente ist ein Pyrimidin-Derivat gemäß Anspruch 1.

Diese Pyrimidin-Derivate, wie beispielsweise 2,4,5,6-Tetraaminopyrimidin, und 4-Hydroxy-2,5,6,-triaminopyrimidin, sind dem Fachmann bekannte Verbindungen. Stellvertretend für eine Vielzahl von Druckschriften, die diese Verbindungen offenbaren, wird auf die DE-OS 23 59 399 verwiesen. Auf den Inhalt dieser Druckschrift, insbesondere soweit er die Herstellung dieser Verbindungen offenbart, wird ausdrücklich Bezug genommen.

Besonders bevorzugte Pyrimidin-Derivate sind, sowohl im alleinigen Einsatz als auch als Kombination, 2,4,5,6-Tetraaminopyrimidin und 4-Hydroxy-2,5,6-triaminopyrimidin.

Anstelle der Pyrimidine gemäß Anspruch 1 kann in den erfindungsgemäßen Färbemitteln als Entwicklerkomponente auch die Verbindung 1-(2'-Hydroxyethyl)-2,5-diaminobenzol verwendet werden. Hinsichtlich dieser Verbindung wird insbesondere auf die Offenbarung der DE-A1-39 17 304 und DE-A1-4400757 verwiesen, die diese Komponente unter der Bezeichnung 2-(2,5-Diaminophenyl)ethanol beschreibt und auch deren Verwendung in Haarfärbemitteln offenbart.

Von der vorliegenden Erfindung ebenfalls umfaßt ist die Verwendung von Kombinationen aus Pyrimidinen gemäß Anspruch 1 und 1-(2'-Hydroxyethyl)-2,5-diaminobenzol als Entwicklerkomponente in Färbemittel.

Die in den erfindungsgemäßen Mitteln enthaltenen Entwickler- und Kupplerkomponenten können sowohl als freie Basen als auch in Form ihrer Salze mit anorganischen oder organischen Säuren, z.B. der Hydrochloride, der Sulfate und Hydrobromide, eingesetzt werden.

Die erfindungsgemäßen Haarfärbemittel enthalten sowohl die Entwicklerkomponenten als auch die Kupplerkomponenten bevorzugt in einer Menge von 0,01 bis 20 Gew.-%, vorzugsweise 0,5 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten bevorzugt in einem Mol-Verhältnis von 1 : 0,5 bis 1 : 2 enthalten sein können.

Neben den erfindungsgemäßen Kuppler-/Entwickler-Kombinationen können die Haarfärbemittel gewünschtenfalls weitere Kuppler- und/oder Entwicklerkomponenten enthalten, um spezielle Farbnuancen zu erhalten.

Bevorzugte weitere Kupplerkomponenten werden insbesondere ausgewählt aus
- Resorcin und Resorcin-Derivaten,
- Naphthalin-Derivaten mit mindestens einer OH-Gruppe sowie
- Pyridin-Derivaten mit mindestens einer C₁₋₄-Alkylgruppe und mindestens einer Hydroxygruppe.

Besonders geeignete weitere Kupplerkomponenten sind Resorcin, 2-Methylresorcin, 5-Methylresorcin, 2,5-Dimethylresorcin, 4-Chlorresorcin, 1,5-, 1,7- und 2,7-Dihydroxynaphthalin sowie 2,6-Dihydroxy-3,4-dimethylpyridin. 2-Methylresorcin, Resorcin sowie 2,7-Dihydroxynaphthalin sind ganz besonders bevorzugt.

Weitere, in den erfindungsgemäßen Mitteln enthaltene Entwicklerkomponenten werden bevorzugt ausgewählt aus 1,4-Diaminobenzol, 3-Methyl-1,4-diaminobenzol, 2,5-Diaminotoluol, 3-Methyl-4-aminophenol, 2-Methylamino-4-aminophenol und N,N-Bis(2'-Hydroxyethyl)-1,4-diaminobenzol.

In einer weiteren bevorzugten Ausführungsform enthalten die erfindungsgemäßen Haarfärbemittel zur weiteren Modifizierung der Farbnuancen neben den Oxidationsfarbstoffvorprodukten zusätzlich übliche direktziehende Farbstoffe, z.B. aus der Gruppe der Nitrophenylendiamine, Nitroaminophenole, Anthrachinone oder Indophenole, wie z.B. die unter den internationalen Bezeichnungen bzw. Handelsnamen HC Yellow 2, HC Yellow 4, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16, Basic Brown 17, Pikraminsäure und Rodol 9 R bekannten Verbindungen sowie 4-Amino-2-nitrodiphenylamin-2'-carbonsäure, 6-Nitro-1,2,3,4-tetrahydrochinoxalin, (N-2,3-Dihydroxypropyl-2-nitro-4-trifluormethyl)aminobenzol und 4-N-Ethyl-1,4-bis(2'-hydroxyethylamino)-2-nitrobenzol-hydrochlorid in einer Menge von 0,01 bis 20 Gew.-%, bezogen auf das gesamte Oxidationshaarfärbemittel.

Weiterhin können die erfindungsgemäßen Färbemittel auch in der Natur vorkommende Farbstoffe wie beispielsweise Henna rot, Henna neutral, Henna schwarz, Kamillenblüte, Sandelholz, schwarzer Tee, Faulbaumrinde, Salbei, Blauholz, Krappwurzel, Catechu, Sedre und Alkannawurzel enthalten.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Zur Herstellung der erfindungsgemäßen Färbemittel werden die Oxidationsfarbstoffvorprodukte in einen geeigneten wasserhaltigen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Die erfindungsgemäßen Färbemittel werden bevorzugt auf einen pH-Wert von 6,5 bis 11,5 insbesondere von 9 bis 10, eingestellt.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Anionische Tenside können dabei ganz besonders bevorzugt sein.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ether-, Amid- und Hydroxylgruppen sowie in der Regel auch Estergruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare und verzweigte Fettsäuren mit 8 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen, - Alkylsulfate und Alkylpolyglykolethersulfate der Formel R-O(CH₂-CH₂O)ₓ-OSO3H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylen glykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungs produkte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C8-C22-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethyl-ammoniumglycinate, beispielsweise das Kokosacylaminopropyl-dimethylammonium-glycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethyl-hydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C8-C18-Alkyl- oder -Acylgruppe im Molekül mindestens eine freie Aminogruppe und mindestens eine -COOH- oder -SO3H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C 12-18-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C 12-C22-Fettsäuremono- und -diester von Anlagerungsprodukten von 1 bis 30 Mol Ethylenoxid an Glycerin,
- C8-C22-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester,
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{R}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{R}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{R} vertriebenen Dialkylammoniummethosulfate und Methyl-hydroxyalkyl-dialkoyloxyalkyl-ammoniummethosulfate.

Ein Beispiel für ein als kationisches Tensid einsetzbares quaternäres Zuckerderivat stellt das Handelsprodukt Glucquat^{R}100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert. Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Farbstoffe zum Einfärben der Zubereitungen, Antischuppenwirkstoffe wie Piroctone Olamine und Zink Omadine,
- Alkalisierungsmittel wie beispielsweise Ammoniak, Monoethanolamin, 2-Amino-2-methylpropanol und 2-Amino-2-methyl-propandiol-1,3.
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether, -
   Fette und Wachse wie Walrat, Bienenwachs, Montanwachs, Paraffine,
   Fettalkohole und Fettsäureester,
   Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N2O, Dimethylether, CO2 und Luft,
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0, 1 bis 25 Gew.-% des gesamten Färbemittels eingesetzt.

Die oxidative Entwicklung der Färbung kann grundsätzlich mit Luftsauerstoff erfolgen. Dieses Verfahren kann insbesondere dann bevorzugt sein, wenn die Verbindungen gemäß Formel (II) die alleinigen Entwicklerkomponenten innerhalb des Mittels sind. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen insbesondere Wasserstoffperoxid oder dessen Anlagerungsprodukte an Harnstoff, Melamin oder Natriumborat. Weiterhin ist es möglich, die Oxidation mit Hilfe von Enzymen durchzuführen. Dabei können die Enzyme sowohl zur Erzeugung von oxidierenden Per-Verbindungen eingesetzt werden, als auch zu Verstärkung der Wirkung einer geringen Mengen vorhandener Oxidationsmittel. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die nachfolgenden Beispiele sollen den Erfindungsgegenstand näher erläutern.

### Beispiele

Es wurde zunächst eine Cremebasis folgender Zusammensetzung hergestellt [alle Angaben sind, soweit nicht anders vermerkt, in g]:

| | |
|---|---|
| Talgfettalkohol | 17,0 |
| Lorol^{®}techn.¹ | 4,0 |
| Texapon^{®}N 28² | 40,0 |
| Dehyton^{®}K³ | 25,0 |
| Eumulgin^{®}B 2⁴ | 1,5 |
| destilliertes Wasser | 12,5 |

| | |
|---|---|
| ¹ C₁₂-₁₈-Fettalkohol (HENKEL) ² Natriumlaurylethersulfat (ca. 28 % Aktivsubstanz; CTFA-Bezeichnung: Sodium Laureth Sulfate) (HENKEL) ³ Fettsäureamid-Derivat mit Betainstruktur der Formel R-CONH(CH₂)₃N⁺(CH₃)₂CH₂COO- (ca. 30 % Aktivsubstanz; CTFA-Bezeichnung Cocoamidopropyl Betaine) (HENKEL) ⁴ Cetylstearylalkohol mit ca. 20 Mol EO (CTFA-Bezeichnung: Ceteareth-20)° (HENKEL) | |

Auf Basis dieser Creme wurde dann folgende Haarfärbecremeemulsion hergestellt:

| | |
|---|---|
| Cremebasis | 50,0 |
| Entwicklerkomponente | 7,5 mmol |
| Kupplerkomponente | 7,5 mmol |
| Na₂SO₃(Inhibitor) | 1,0 |
| (NH₄)₂SO₄ | 1,0 |
| konz. Ammoniaklösung | ad pH 10 |
| Wasser | ad 100 |

Die Bestandteile wurden der Reihe nach miteinander vermischt. Nach Zugabe der Oxidationsfarbstoffvorprodukte und des Inhibitors wurde zunächst mit konzentrierter Ammoniaklösung der pH-Wert der Emulsion auf 10 eingestellt, dann wurde mit Wasser auf 100 g aufgefüllt.

Die oxidative Entwicklung der Färbung wurde mit Wasserstoffperoxidlösung als Oxidationslösung durchgeführt. Hierzu wurden 100 g der Emulsion mit 50 g Wasserstoffperoxidlösung (1,3 oder 9 %ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 32 °C belassen. Nach Beendigung des Färbeprozesses wurde das Haar gespült, mit einem üblichen Haarwaschmittel gewaschen und anschließend getrocknet.

Die Ausfärbung nur mit Luftsauerstoff erfolgte analog ohne Zugabe des Oxidationsmittels.

Für die Ausfärbungen wurden folgende Kuppler- und Entwickler-Komponenten verwendet:
- Kuppler-Komponenten :
   2,6-Bis(2-hydroxyethylamino)toluol (K1)
   2,7-Dihydroxynaphthalin (K2)
- Entwickler-Komponenten:
   2,4,5,6-Tetraaminopyrimidin (E1)
   4-Hydroxy-2,5,6-Triaminopyrimidin (E2)
   2-(2-Hydroxyethyl)-1,4-diaminobenzol(E7)
   2,5-Dianünotoluot(E8) (nicht erfindungsgemässe)

### Direktziehender Farbstoff:

### 4-(3'-Trimethylammoniumphenylazo)-1-phenyl-3-methyl-pyrazolon-chlorid (D1)

Es wurden folgende Ausfärbungen gefunden:

| Kuppler | Entwickler | Oxidations- mittel | direktziehender Farbstoff | Nuance des gefärbten Haares |
|---|---|---|---|---|
| K1 | E1 | 3 % H₂O₂ | - | tiefrot |
| K1 | E2 | 3 % H₂O₂ | - | braunrot |
| | | | - | |
| K1 +K2 | E1 | 3 % H₂O₂ | - | kupferfarben |
| K1 | E7 | Luft | - | grauviolett |
| K1 | E7 | 1 % H₂O₂ | - | violett |
| K1 | E7 | 9 % H₂O₂ | - | violett |
| K1 | E1⁺+E8⁺ | Luft | - | graurosa |
| K1 | E1⁺+E8⁺ | 1 % H₂O₂ | - | graurubin |
| K1 | E1⁺+E8⁺ | 9 % H₂O₂ | - | tiefmagenta |

| | | | | |
|---|---|---|---|---|
| * 2,5 mmol ⁺ 1,25mmol | | | | |

Mit einer Färbecremeemulsion wie oben angegeben, die als Färbekomponenten
- 2,50 g E-1 (als Salz mit einem Molekül Schwefelsäure pro Pyrimidin-Derivat)
- 0,25 g E-7
- 0,08 g 3-Methyl-p-aminophenol
- 0,02 g K-1
- 0,42 g Resorcin
- 0,76 g 2-Methylresorcin
- 0,14 g 3-Amino-2-methylamino-6-methoxypyridin-dihydrochlorid und
- 0,05 g 2-Amino-6-chlor-4-nitrophenol
enthielt, wurde eine rotbraune Ausfärbung der Haare erzielt.

Mit einer Färbecremeemulsion wie oben angegeben, die als Färbekomponenten
- 1,95 g E-1 (als Salz mit einem Molekül Schwefelsäure pro Pyrimidin-Derivat)
- 0,33 g K-1
- 0,12 g 2-Methylresorcin und
- 0,80 g K-2
enthielt, wurde eine leuchtend kupfertonfarbene Ausfärbung der Haare erzielt.

## Patentansprüche

1. Oxidationsfärbemittel zum Färben von Keratinfasern enthaltend mindestens eine Kupplerkomponente und mindestens eine Entwicklerkomponente in einem wasserhaltigen Träger, **dadurch gekennzeichnet, dass**
- die Kupplerkomponente ausgewählt ist aus den Verbindungen gemäß Formel (I), 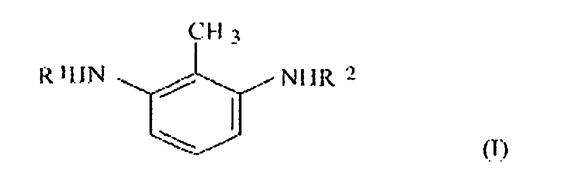 in der R¹ und R² unabhängig voneinander stehen für Wasserstoff, eine Mono-oder Dihydroxyalkylgruppe mit 2 bis 4 Kohlenstoffatomen oder eine Allylgruppe mit der Maßgabe, dass R¹ und R² nicht gleichzeitig Wasserstoff sind, und deren
Salzen mit einer anorganischen oder organischen Säure und
- die Entwicklerkomponente ausgewählt ist aus 2,4,5,6-Tetraamino-pyrimidin und 4-Hydroxy-2,5,6-triaminopyrimidin und deren Salzen mit einer anorganischen oder organischen Säure und/oder 1-(2'-Hydroxyethyl)-2,5-diaminobenzol und dessen Salzen mit anorganischen und organischen Säuren.

2. Oxidationsfärbemittel nach Anspruch 1, **dadurch gekennzeichnet, dass** mindestens eine der beiden Gruppen R¹ und R² steht für eine 2-Hydroxycthylgruppe, eine 3-Hydroxypropylgruppe oder eine 2,3-Dihydroxypropylgruppe.

3. Oxidationsfärbemittel nach Anspruch 2, **dadurch gekennzeichnet, dass** die Kupplerkomponente ausgewählt ist aus 2,6-Bis(2'-hydroxyethylamino)-toluol und 2,6-Bis(3'-hydroxypropylamino)-toluol.

4. Oxidationsfärbemittel nach einem der Ansprüche 1 bis 3, **dadurch gekennzeichnet**, das die Kupplerkomponenten in einer Menge von 0,01 bis 20 Gew.-%, und die Entwicklerkomponenten in einer Menge von 0,01 bis 20 Gew.-% jeweils bezogen auf das gesamte Oxidationsfärbemittel, enthalten sind.

5. Oxidationsfärbemittel nach einem der Ansprüche 1 bis 4, **dadurch gekennzeichnet. dass** mindestens eine weitere Kupplerkomponente und/oder mindestens eine weitere Entwicklerkomponente enthalten ist.

6. Oxidationsfärbemittel nach Anspruch 5, **dadurch gekennzeichnet, dass** die weitere Kupplerkomponente ausgewählt ist aus
- Resorcin und Resorcin-Derivaten,
- Naphthalin-Derivaten mit mindestens einer OH-Gruppe sowie
- Pyridin-Derivaten mit mindestens einer C₁₋₄-Alkylgruppe und mindestens einer Hydroxygruppe.

7. Oxidationsfärbemittel nach Anspruch 6, **dadurch gekennzeichnet, dass** die weitere Kupplerkomponente ausgewählt ist aus Resorcin, 2-Methylresorein, 5-Methylresorcin. 2.5-Dimethylresorein, 4-Chlorresorein, 1,5-, 1,7- und 2,7-Dihydroxynaphthalin sowie 2,6-Dihydroxy-3,4-dimethylpyridin.

8. Oxidationsfärbemittel nach einem der Ansprüche 5 bis 7, **dadurch gekennzeichnet**, das die weitere Entwicklerkomponente ausgewählt ist aus 1,4-Diaminobenzol, 3-Methyl-1,4-diaminobenzol, 2,5-Diaminotoluol, 3-Methyl-4-aminophenol, 2-Methyl-amino-4-aminophenol und N,N-Bis(2'-Hydroxyethyl)-1,4-diaminobenzol.

9. Oxidationsfärbemittel nach einem der Ansprüche 1 bis 8, **dadurch gekennzeichnet**, das mindestens ein direktzichender Farbstoff enthalten ist.

10. Verwendung eines Mittels nach einem der Ansprüche 1 bis 9 zum Färben von menschlichen Haaren.

## Claims

1. Oxidation colourant for dyeing keratin fibres comprising at least one coupler component and at least one developer component in a hydrous carrier, **characterized in that**
- the coupler component is selected from the compounds according to formula (I), 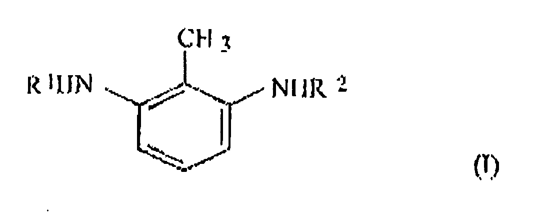
in which R¹ and R², independently of one another, are hydrogen, a mono- or dihydroxyalkyl group having 2 to 4 carbon atoms or an allyl group with the proviso that R¹ and R² are not hydrogen at the same time, and salts thereof with an inorganic or organic acid and
- the developer component is selected from 2,4,5,6-tetraaminopyrimidine and 4-hydroxy-2,5,6-triaminopyrimidine and salts thereof with an inorganic or organic acid and/or 1-(2'-hydroxyethyl)-2,5-diaminobenzene and salts thereof with inorganic and organic acids.

2. Oxidation colourant according to Claim 1,
**characterized in that** at least one of the two groups R¹ and R² is a 2-hydroxyethyl group, a 3-hydroxypropyl group or a 2,3-dihydroxypropyl group.

3. Oxidation colourant according to Claim 2,
**characterized in that** the coupler component is selected from 2,6-bis(2'-hydroxyethylamino)toluene and 2,6-bis(3'-hydroxypropylamino)toluene.

4. Oxidation colourant according to one of Claims 1 to 3, **characterized in that** the coupler components are present in an amount of from 0.01 to 20% by weight, and the developer components are present in an amount of from 0.01 to 20% by weight, in each case based on the total oxidation colourant.

5. Oxidation colourant according to one of Claims 1 to 4, **characterized in that** at least one further coupler component and/or at least one further developer component is present.

6. Oxidation colourant according to Claim 5,
**characterized in that** the further coupler component is selected from
- resorcinol and resorcinol derivatives,
- naphthalene derivatives with at least one OH group, and
- pyridine derivatives with at least one C₁₋₄-alkyl group and at least one hydroxy group.

7. Oxidation colourant according to Claim 6, **characterized in that** the further coupler component is selected from resorcinol, 2-methylresorcinol, 5-methylresorcinol, 2,5-dimethylresorcinol, 4-chlororesorcinol, 1,5-, 1,7- and 2,7-dihydroxynaphthalene and 2,6-dihydroxy-3,4-dimethylpyridine.

8. Oxidation colourant according to one of Claims 5 to 7, **characterized in that** the further developer component is selected from 1,4-diaminobenzene, 3-methyl-1,4-diaminobenzene, 2,5-diaminotoluene, 3-methyl-4-aminophenol, 2-methylamino-4-aminophenol and N,N-bis(2'-hydroxyethyl)-1,4-diaminobenzene.

9. Oxidation colourant according to one of Claims 1 to 8, **characterized in that** at least one direct dye is present.

10. Use of a composition according to one of Claims 1 to 9 for dyeing human hair.

## Revendications

1. Teinture d'oxydation pour la teinture de fibres kératiniques, contenant au moins un composant faisant office de coupleur et au moins un composant faisant office de développeur dans un support aqueux,
**caractérisée en ce que** :
- le composant faisant office de coupleur est choisi parmi les composés répondant à la formule (I) 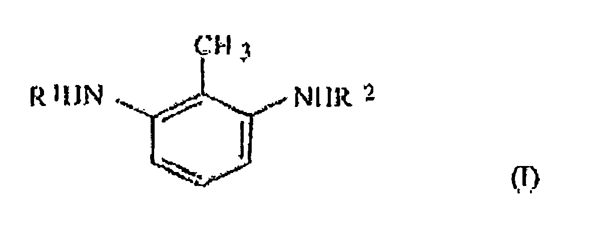
dans laquelle R¹ et R² représentent, indépendamment l'un de l'autre, un atome d'hydrogène, un groupe mono- ou dihydroxyalkyle contenant de 2 à 4 atomes de carbone ou un groupe allyle, avec cette mesure que R¹ et R² ne représentent pas en même temps un atome d'hydrogène, ainsi que leurs sels avec un acide inorganique ou organique, et
- le composant faisant office de développeur est choisi parmi la 2,4,5,6-tétra-amino-pyrimidine et la 4-hydroxy-2,5,6-triaminopyrimidine et leurs sels avec un acide inorganique ou organique et/ou le 1-(2'-hydroxyéthyl)-2,5-diaminobenzène et ses sels avec des acides inorganiques et organiques.

2. Teinture d'oxydation selon la revendication 1, **caractérisée en ce qu'**au moins un des deux groupes R¹ et R² représente un groupe 2-hydroxyéthyle, un groupe 3-hydroxypropyle ou un groupe 2,3-dihydroxypropyle.

3. Teinture d'oxydation selon la revendication 2, **caractérisée en ce que** le composant faisant office de coupleur est choisi parmi le 2,6-bis(2'-hydroxyéthylamino)-toluène et le 2,6-bis(3'-hydroxypropylamino)-toluène.

4. Teinture d'oxydation selon l'une quelconque des revendications 1 à 3, **caractérisée en ce que** les composants faisant office de coupleurs sont contenus en une quantité de 0,01 à 20 % en poids et les composants faisant office de développeurs sont contenus en une quantité de 0,01 à 20 % en poids, chaque fois rapportés à la teinture d'oxydation total.

5. Teinture d'oxydation selon l'une quelconque des revendications 1 à 4, **caractérisée en ce qu'**elle contient au moins un composant supplémentaire faisant office de coupleur et/ou au moins un composant supplémentaire faisant office de développeur.

6. Teinture d'oxydation selon la revendication 5, **caractérisée en ce que** le composant supplémentaire faisant office de coupleur est choisi parmi :
- le résorcinol et des dérivés du résorcinol
- des dérivés du naphtalène comprenant au moins un groupe OH, et
- des dérivés de la pyridine comprenant au moins un groupe alkyle en C₁-C₄ et au moins un groupe hydroxyle.

7. Teinture d'oxydation selon la revendication 6, **caractérisée en ce que** le composant supplémentaire faisant office de coupleur est choisi parmi le résorcinol, le 2-méthylrésorcinol, le 5-méthylrésorcinol, le 2,5-diméthylrésorcinol, le 4-chlororésorcinol, le 1,5-, le 1,7- et le 2,7-dihydroxynaphtalène, ainsi que la 2,6-dihydroxy-3,4-diméthylpyridine.

8. Teinture d'oxydation selon l'une quelconque des revendications 5 à 7, **caractérisée en ce que** le composant supplémentaire faisant office de développeur est choisi parmi le 1,4-diaminobenzène, le 3-méthyl-1,4-diaminobenzène, le 2,5-diaminotoluène, le 3-méthyl-4-aminophénol, le 2-méthylamino-4-aminophénol et le N,N-bis(2'-hydroxyéthyl)-1,4-diaminobenzène.

9. Teinture d'oxydation selon l'une quelconque des revendications 1 à 8, **caractérisée en ce qu'**elle contient au moins un colorant montant directement sur la fibre.

10. Utilisation d'un agent selon l'une quelconque des revendications 1 à 9 pour la teinture de cheveux humains.
